# EUROPEAN PATENT APPLICATION

(11) **EP 4 579 228 A1**
(43) Date of publication of application: **02.07.2025**
(21) Application number: 23845056.3
(22) Date of filing: 30.05.2023
(51) Int. Cl.: G01N 27/26, A61B 5/1486, A61B 5/145, A61B 5/1468

(54) **SENSOR FOR MONITORING LACTIC ACID, AND MANUFACTURING METHOD THEREFOR**

(30) Priority: 24.07.2022 CN 202210874355
(71) Applicant: SHENZHEN SISENSING CO., LTD., Shenzhen Guangdong 518110 (CN)
(72) Inventor: LIU, Qiaoqiao, Shenzhen, Guangdong 518110 (CN); FANG, Junfei, Shenzhen, Guangdong 518110 (CN); CHEN, Liguo, Shenzhen, Guangdong 518110 (CN); LAI, Ming, Shenzhen, Guangdong 518110 (CN)
(74) Representative: Isern Patentes y Marcas S.L.
(86) International application number: PCT/CN2023/097271
(87) International publication number: WO 2024/021843

(57) **Abstract**

The present disclosure relates to a sensor (1) for monitoring lactic acid, which includes a working electrode (12), an enzyme sensing layer (13), and a semi-permeable membrane (14). The working electrode (12) is formed by a nano slurry, which is a conductive slurry having nanoparticles. The enzyme sensing layer (13) is provided on the working electrode (12), the enzyme sensing layer (13) includes a lactic acid enzyme and is capable of reacting with lactic acid. The semi-permeable membrane (14) is provided on the enzyme sensing layer (13) and at least covers the enzyme sensing layer (13), the semi-permeable membrane (14) includes a first membrane layer (141) formed by a first membrane solution, wherein a solute of the first membrane solution includes polyvinyl pyridine and a solvent is pure ethanol. A sensor (1) for monitoring lactic acid with high sensitivity and good stability for monitoring lactic acid can be provided according to the present disclosure. In addition, the present disclosure also provides a method for preparing the above-mentioned sensor (1) for monitoring lactic acid.

## Description

### Background of invention

### Field of Invention

The present disclosure relates substantially to the field of biosensor, specifically to a sensor for monitoring lactic acid and preparation method thereof.

### Description of Related Art

Lactic acid, as an important metabolic substance in the human body, has significant physiological significance in terms of its concentration in the blood. Generally speaking, when the energy requirements of tissues cannot be met through aerobic respiration (for example, due to insufficient oxygen supply or low oxygen processing rate), the concentration of lactic acid in the human body will increase. Typically, the concentration of lactic acid in the blood ranges from 1 mmol/L to 2mmol/L at rest, and it can rise to 10mmol/L during exercise, and even up to 25mmol/L during intense exercise. Therefore, monitoring the concentration of lactic acid in the blood of the human body, especially for athletes, is of great significance. On the other hand, lactic acid concentration monitoring can also be applied in the medicine field, such as in cases where the lactic acid level rises after a person is infected with novel coronavirus, or in patients with conditions like shock or sepsis, where the lactic acid level can also increase in some individuals.

In recent years, the demand for medical devices related to in vivo lactic acid concentration monitoring for clinical diagnosis and personal care has increased exponentially. Convenient, rapid, and accurate lactic acid monitoring devices have flourished and attracted widespread attention, especially implantable continuous lactic acid monitoring devices, which have been favored by athletes and major hospitals.

With the development of technology, many lactic acid sensors have emerged that catalyze lactic acid reactions through lactic acid responsive enzymes and redox electron mediators and perform electrochemical detection. However, the current lactic acid sensors exhibit low sensitivity, and their stability during continuous monitoring needs further improvement.

### Summary of invention

The present disclosure is made in view of above-mentioned situation, and its object is to provide a sensor for monitoring lactic acid with high sensitivity and good stability, as well as its preparation method.

For the above purposes, a sensor for monitoring lactic acid is provided according to the first aspect of the present disclosure, which includes a working electrode, an enzyme sensing layer, and a semi-permeable membrane. The working electrode is formed by a nano slurry, wherein the nano slurry is a conductive slurry having nanoparticles. The enzyme sensing layer is provided on the working electrode, the enzyme sensing layer includes a lactic acid enzyme and is capable of reacting with the lactic acid. The semi-permeable membrane is provided on the enzyme sensing layer and at least covers the enzyme sensing layer, the semi-permeable membrane includes a first membrane layer formed by a first membrane solution, wherein a solute of the first membrane solution includes polyvinyl pyridine and a solvent is pure ethanol.

In the first aspect of the present disclosure, the working electrode is formed by the nano slurry, wherein the nano slurry is a conductive slurry having nanoparticles. the nanoparticles are added into the working electrode, thereby the conductivity of the working electrode can be increased, resulting in improving the sensitivity of the sensor; The semi-permeable membrane includes a first membrane layer formed by a first membrane solution, the solute of the first membrane solution includes polyvinyl pyridine and the solvent is pure ethanol, the solvent of the first membrane solution is provided to be pure ethanol, thereby the formation of the first membrane layer can be facilitated with good morphological consistency, dense morphology, and restricted permeability, resulting in facilitating the improvement of stability of the sensor. Thus, a sensor for monitoring lactic acid with high sensitivity and good stability can be provided.

In addition, in the senor according to the first aspect of the present disclosure, optionally, the enzyme sensing layer is formed by a lactic acid-sensitive solution, which includes lactic acid enzyme, serum albumin, metal polymer, and crosslinking agent. In this case, the serum albumin is added into the enzyme sensing layer, thereby facilitating to keep the activity of the lactic acid enzyme, and to improve the stability of the lactic acid reaction, resulting in facilitating the improvement of the stability of the sensor; The metal polymer is added into the enzyme sensing layer, thereby the catalysis of the redox reaction of lactic acid can be facilitated, resulting in improving the sensitivity of the sensor against lactic acid reactions.

In addition, in the senor according to the first aspect of the present disclosure, optionally, the metal polymer is selected from at least one of poly(vinylferrocene), quaternary ammonium poly(4-vinylpyridine) of ferricyanide, quaternary ammonium poly(1-vinylimidazole) of ferricyanide, quaternary ammonium poly(4-vinylpyridine) of ferrocyanide, quaternary ammonium poly(1-vinylimidazole) of ferrocyanide, osmium bipyridyl complex coordinated to poly(1-vinylimidazole), osmium bipyridyl complex coordinated to poly(4-vinylpyridine), cobalt bipyridyl complex coordinated to poly(1-vinylimidazole), or cobalt bipyridyl complex coordinated to poly(4-vinylpyridine). Thus, the metal polymer is capable of participating in redox reactions through covalent bonds, coordination bonds, or ionic bonds.

In addition, in the senor according to the first aspect of the present disclosure, optionally, the lactic acid-sensitive solution is drop-coated onto the working electrode in the form of a plurality of sensing spots to form the enzyme sensing layer, diameters of the sensing spots range from 80µm to 170µm, and the number of sensing spots is 6 to15. In this case, the area and number of sensing spots are adjusted, thereby the adjustment of the contact area between the enzyme sensing layer and the working electrode can be facilitated, resulting in facilitating the adjustment of the magnitude of electrode current.

In addition, in the senor according to the first aspect of the present disclosure, optionally, the concentration of polyvinyl pyridine in the first membrane solution ranges from 100mg/mL to 150mg/mL. Thereby, the formation of the first membrane layer having limited permeation effect can be facilitated.

In addition, in the senor according to the first aspect of the present disclosure, optionally, the molecular weight of polyvinyl pyridine ranges from 10000Da to 500000Da. Thereby, the improvement of the membrane-forming properties of the first membrane layer can be facilitated.

In addition, in the senor according to the first aspect of the present disclosure, optionally, the semi-permeable membrane further includes a second membrane layer having biocompatibility, the second membrane layer is formed by a second membrane solution, a solute of the second membrane solution includes polyvinyl-pyridine-co-polystyrene, and a solvent is pure ethanol. In this case, the polyvinyl-pyridine-co-polystyrene is added into the second membrane layer, the improvement of mechanical strength and biocompatibility of the second membrane layer can be facilitated, the solvent of the second membrane solution is provided to be pure ethanol, the morphological consistency of the second membrane layer can be improved, resulting in facilitating further improvement of the stability of the sensor

In addition, in the senor according to the first aspect of the present disclosure, optionally, the concentration of polyvinyl-pyridine-co-polystyrene in the second membrane solution ranges from 50mg/mL to 150mg/mL. Thereby, further improvement of mechanical strength and biocompatibility of the second membrane layer can be facilitated

In addition, in the senor according to the first aspect of the present disclosure, optionally, the second membrane layer is provided on the first membrane layer, and the thickness ratio of the first membrane layer with the second membrane layer ranges from 1.5:1 to 2.5:1. In this case, the improvement of the adhesion stability between the first membrane layer and the second membrane layer can be facilitated, resulting in facilitating the stability and morphological consistency of the semi-permeable membrane.

A preparation method of the sensor for monitoring lactic acid is provided according to the second aspect of the present disclosure, which includes: Preparing a working electrode, wherein the working electrode is formed by a nano slurry, which is a conductive slurry having nanoparticles; Providing an enzyme sensing layer on the working electrode, wherein the enzyme sensing layer includes lactic acid enzyme and is capable of reacting with lactic acid; Forming a semi-permeable membrane on the enzyme sensing layer, the semi-permeable membrane at least covers the enzyme sensing layer and includes a first membrane layer formed by a first membrane solution, wherein a solute of the first membrane solution includes polyvinyl pyridine and a solvent is pure ethanol. In the second aspect of the present disclosure, the preparation method is used to prepare a sensor for monitoring lactic acid, thereby a sensor with high sensitivity and good stability for monitoring lactic acid can be obtained.

According to the present disclosure, a sensor with high sensitivity and good stability for monitoring lactic acid and the preparation method thereof can be provided.

### Brief description of the drawings

Fig.1 is a schematic diagram showing the usage status of the sensor according to the present disclosure.
Fig.2 is a schematic structural diagram showing the sensor according to the present disclosure.
Fig.3 is a schematic diagram showing another embodiment of the sensor according to the present disclosure.
Fig.4 is a flowchart showing the preparation of the sensor according to the present disclosure.
Fig.5 is a graph showing the current and temperature response-test time of the sensor according to embodiment 1 of the present disclosure.
Fig.6 is a graph showing the current and temperature response-test time of the sensor according to embodiment 2 of the present disclosure.
Fig.7 is a graph showing the current and temperature response-test time of the sensor according to embodiment 3 of the present disclosure.
Fig.8 is a graph showing the current and temperature response-test time of the sensor according to comparative example 1 of the present disclosure.
Fig.9 is a graph showing the current and temperature response-test time of the sensor according to comparative example 2 of the present disclosure.
Fig.10 is a photograph showing the monitoring probe of the sensor according to comparative example 2 of the present disclosure.

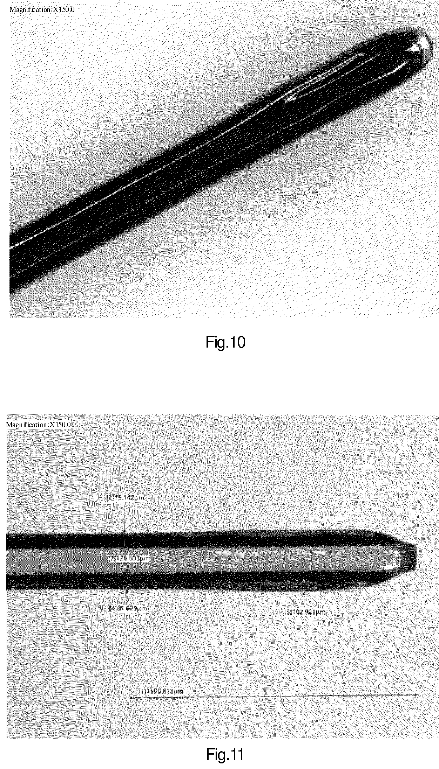

Fig.10 is another photograph showing different view of the monitoring probe of the sensor according to comparative example 2 of the present disclosure.

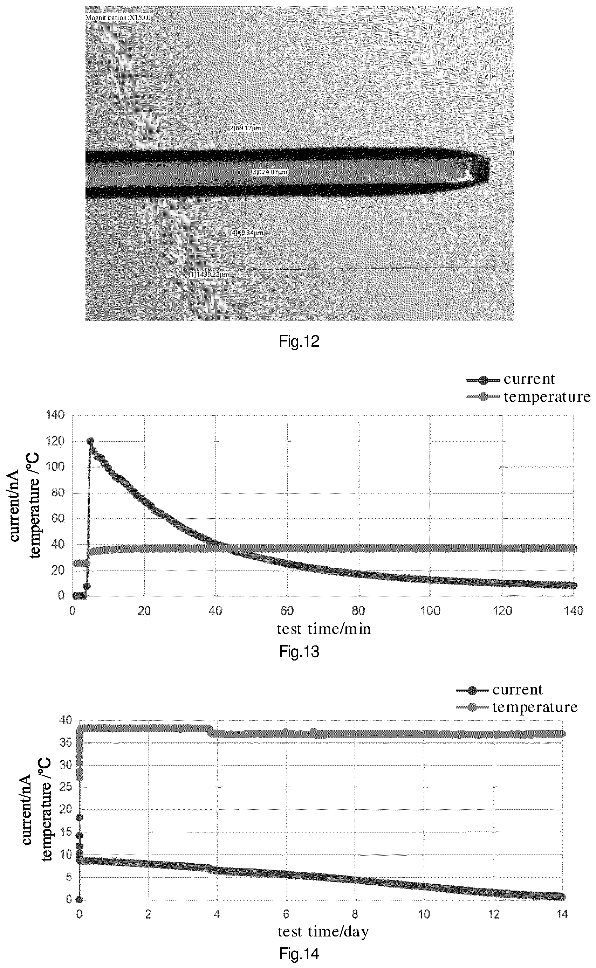

Fig.12 is a photograph showing the monitoring probe of the sensor according to embodiment 1 of the present disclosure.
Fig.13 is a graph showing the current and temperature response-test time of the sensor according to comparative example 3 of the present disclosure.
Fig.14 is a graph showing the current and temperature response-test time of the sensor according to comparative example 4 of the present disclosure.

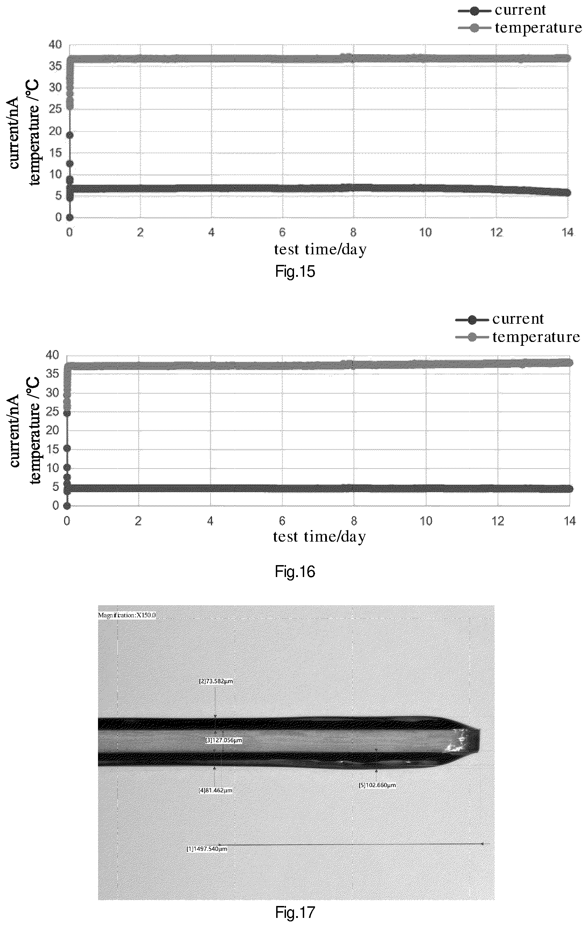

Fig.15 is a graph showing the current and temperature response-test time of the sensor according to comparative example 5 of the present disclosure.
Fig.16 is a graph showing the current and temperature response-test time of the sensor according to comparative example 6 of the present disclosure.
Fig.17 is a photograph showing the monitoring probe of the sensor according to comparative example 6 of the present disclosure.

### Description for markings of the accompany drawings:

1...Sensor, 10...Monitoring probe, 11...Substrate, 12...Working electrode, 13...Enzyme sensing layer, 131...sensing points 14...Semi-permeable membrane, 141...First membrane layer, 142...Second membrane layer, 15...Reference electrode, 16...Counter electrode, 17...Insulating layer, 20... Electronic system.

### Detailed description

All references cited in the present disclosure are hereby introduced in full for reference, as if fully set forth herein. Unless otherwise defined, the technical and scientific terms used in the present disclosure have the same meanings as commonly understood by an ordinary person skilled in the art to which the present disclosure belongs.

Hereinafter, preferred embodiments of the present disclosure will be described in detail with reference to the accompanying drawings. In the following descriptions, identical components are assigned the same symbols, and redundant explanations are omitted. In addition, the accompany drawings are merely schematic diagrams, and the proportions of the sizes or shapes of the components relative to each other may differ from actual implementations.

It should be noted that the terms "include" and "have", and any variations thereof in the present disclosure, such as a process, method, system, product, or equipment that includes or has a series of steps or elements are not necessarily limited to those explicitly recited steps or elements, but may include or have other steps or elements not explicitly recited or inherent to such process, method, product, or equipment.

The first aspect of the present disclosure relates to a sensor for monitoring lactic acid, which exhibits high sensitivity and good stability. In the present disclosure, the sensor for monitoring lactic acid may be referred to as a "lactic acid monitoring sensor", "lactic acid sensor", "physiological parameter sensor", or simply "sensor", the preparation method of the sensor for monitoring lactic acid may be referred to as "preparation method".

Hereinafter, the sensor for monitoring lactic acid will be described in combination with the accompanying drawings according to the first aspect of the present disclosure.

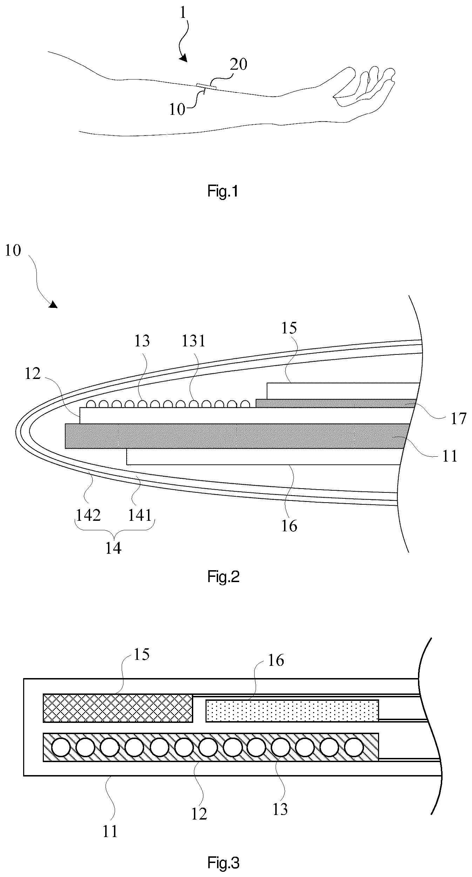

Fig.1 is a schematic diagram showing the usage status of the sensor 1 according to the present disclosure.

In the present embodiment, sensor 1 may include monitoring probe 10 and electronic system 20. In some examples, monitoring probe 10 may be implanted on the surface of the body, such as the human, and in contact with the tissue fluid within the body, thereby the lactic acid concentration signal of the tissue fluid can be sensed and detected. In some examples, monitoring probe 10 may be connected to electronic system 20. In this case, monitoring probe 10 is in contact with the tissue fluid or blood of the human body, which enabling sensor 1 to sense and detect the signals related to lactic acid concentration in the tissue fluid. The lactic acid concentration signals are transmitted to the electronic system 20, thereby the corresponding lactic acid concentration can be obtained. Although Fig.1 illustrates the configuration position of sensor 1, the present embodiment is not limited to hereof, for example, sensor 1 can also be disposed on the abdomen, waist, legs, etc.

In the present embodiment, sensor 1 may directly inspect lactic acid in the blood, it may also inspect lactic acid in the tissue fluid. In addition, there is a strong correlation between the lactic acid concentration in tissue fluid and the lactic acid concentration in blood, the lactic acid concentration in blood can be determined through the lactic acid concentration in tissue fluid.

Fig.2 is a schematic structural diagram showing sensor 1 according to the present disclosure. The perspective of Fig.2 is a side view. Fig.3 is a schematic diagram showing another embodiment of sensor 1 according to the present disclosure. The perspective of Fig.3 is a top-down view. Wherein, in order to more clearly illustrate the structure of monitoring probe 10 in another embodiment of sensor 1 according to the present disclosure, part of the lines and structures that may cause occlusion are omitted from Fig.3.

In the present embodiment, sensor 1 may include a working electrode 12, an enzyme sensing layer 13, and a semi-permeable membrane 14 (see Fig.2). In some examples, working electrode 12, enzyme sensing layer 13, and semi-permeable membrane 14 may be provided on monitoring probe 10. In this case, monitoring probe 10 is implanted under the skin, thereby sensing and detecting the signals related to the lactic acid concentration in the tissue fluid can be facilitated.

In some examples, working electrode 12 may be formed by a nano slurry. In some examples, the nano slurry is a conductive slurry having nanoparticles. In some examples, the nanoparticles may promote the redox reaction of lactic acid. In this case, the nanoparticles are added into working electrode 12, thereby the conductivity of working electrode 12 can be improved, resulting in improving the sensitivity of sensor 1. In some examples, the nanoparticles may be uniformly distributed in working electrode 12. Thus, the conductivity of working electrode 12 can be further improved.

In some examples, the nanoparticles can be at least one of gold nanoparticles, silver nanoparticles, or platinum nanoparticles. Thus, the promotion of redox reaction of lactic acid can be further facilitated.

In some examples, enzyme sensing layer 13 may be provided on working electrode 12. In some examples, enzyme sensing layer 13 may include lactic acid enzyme. In some examples, enzyme sensing layer 13 may react with lactic acid. Thus, the catalysis of the redox reaction of lactic acid can be facilitated. In some examples, the lactic acid enzyme may be lactic acid oxidase. In some other examples, the lactic acid enzyme may also be lactic acid oxidase.

In some examples, enzyme sensing layer 13 may be formed by lactic acid-sensitive solution. In some examples, the lactic acid-sensitive solution may include lactic acid enzyme. Thus, the catalysis of redox reaction of lactic acid can be facilitated.

In some examples, the lactic acid-sensitive solution may further include serum albumin. Thereby, it is facilitated to keep the biological activity of the lactate oxidase, and the stability of the lactic acid reaction can be improved, resulting in facilitating the improvement of the stability of enzyme sensing layer 13. In some examples, the serum albumin may be bovine serum albumin. In some examples, the serum albumin may also be human serum albumin. Thus, the reduction of the immune response of the human body to sensor 1 can be facilitated, and thereby facilitating further improvement of the stability of enzyme sensing layer 13.

In some examples, the lactic acid-sensitive solution may further include metal polymer. In this case, the metal polymer is added into enzyme sensing layer 13, the catalysis of the redox reaction of lactic acid can be facilitated, resulting in improving the sensitivity of reaction of sensor 1 against lactic acid.

In some examples, the metal polymer may be selected from at least one of poly(vinylferrocene), quaternary ammonium poly(4-vinylpyridine) of ferricyanide, quaternary ammonium poly(1-vinylimidazole) of ferricyanide, quaternary ammonium poly(4-vinylpyridine) of ferrocyanide, quaternary ammonium poly(1-vinylimidazole) of ferrocyanide, osmium bipyridyl complex coordinated to poly(1-vinylimidazole), osmium bipyridyl complex coordinated to poly(4-vinylpyridine), cobalt bipyridyl complex coordinated to poly(1-vinylimidazole), or cobalt bipyridyl complex coordinated to poly(4-vinylpyridine). Thus, the metal polymer is capable of participating in redox reactions through covalent bonds, coordination bonds, or ionic bonds. In some examples, preferably, the metal polymer may be osmium bipyridine complex coordinated to poly(4-vinylpyridine).

In some examples, the lactic acid-sensitive solution may be drop-coated onto working electrode 12 in the form of a plurality of sensing spots 131 to form enzyme sensing layer 13. In some examples, the diameters of sensing spots 131 may range from 80µm to 170µm. In some examples, the number of sensing spots 131 may be 6 to 15. In this case, the area and number of sensing spots 131 is adjusted, the adjustment of the contact area between enzyme sensing layer 13 and working electrode 12 can be facilitated, resulting in facilitating the adjustment of the electrode current. In some examples, preferable, the number of sensing spots 131 may be 12 to 15. For example, the number of sensing spots 131 may be 12, 13, 14, or 15. Thus, improvement of the sensitivity of the sensor 1 can be facilitated.

In some examples, the plurality of sensing spots 131 may form enzyme sensing layer 13 on working electrode 12 in the form of a plurality of discrete points. Thereby, the adjustment of the contact area between enzyme sensing layer 13 and working electrode 12 can be facilitated, resulting in facilitating the adjustment of the electrode current. However, the present disclosure is not limited hereof. In some examples, the edges of the plurality of sensing spots 131 may have overlapping portions. In this case, the edges of the plurality of sensing spots 131 is arranged to be of overlapping portions, arrangement of a greater number of sensing spots 131 on working electrode 12 can be facilitated, resulting in facilitating the improvement of the sensitivity of sensor 1.

In some examples, the plurality of sensing spots 131 may be arranged along the extension direction of working electrode 12. In some examples, the edges of two adjacent sensing spots 131 among the plurality of sensing spots 131 arranged along the extension direction of working electrode 12 may overlapped. In other words, the plurality of sensing spots 131 may be arranged along the extension direction of working electrode 12 to form a substantial line- shape. Thereby, a more efficient utilization of the area of working electrode 12 can be facilitated, resulting in facilitating further improvement of the sensitivity of sensor 1.

In some examples, the lactic acid-sensitive solution may further include cross-linking agent. In some examples, enzyme sensing layer 13 may be formed by coating the lactic acid-sensitive solution onto working electrode 12 and then cross-linked. Thus, the movement of electrons generated by the oxidation-reduction of lactate on working electrode 12 to form electric current can be facilitated.

In some examples, grapheme, porous titanium dioxide, or conductive organic salts may also be added into the lactic acid-sensitive solution. Thus, the reaction of lactic acid enzyme can be preferable promoted.

In some examples, semi-permeable membrane 14 may be provided on enzyme sensing layer 13. In some examples, semi-permeable membrane 14 may at least cover enzyme sensing layer 13. In some examples, semi-permeable membrane 14 may reduce the entering amount of lactic acid. Specifically, semi-permeable membrane 14 may effectively reduce the amount of lactic acid diffusing to enzyme sensing layer 13 by a certain proportion. In this case, semi-permeable membrane 14 is used to at least cover enzyme sensing layer 13, the reduction of amount of lactic acid diffusing to enzyme sensing layer 13 can be facilitated, ensuring that there is a sufficient quantity of lactic acid oxidase or dehydrogenase and other reactants involved in the reaction, and the lactic acid concentration becomes the primary factor limiting the electrode current, thereby enabling the current magnitude to accurately reflect the lactic acid concentration and facilitating the improvement of the linear range of lactic acid concentration monitoring by the sensor 1.

In some examples, the lactic acid-sensitive solution may be used to form enzyme sensing layer 13 by spin coating, dip coating, drop coating, or spray coating. Thus, the selection of an appropriate coating method to form the enzyme sensing layer 13 based on actual needs can be facilitated.

In some examples, the rate at which semi-permeable membrane 14 reduces the amount of substances entering may be 10 to 200 times. Thus, the reduction of the amount of lactic acid diffusing to the enzyme sensing layer 13 can be facilitated. In some examples, preferablely, the rate at which semi-permeable membrane 14 reduces the amount of substances entering may be 100 to 150 times. For example, the rate at which semi-permeable membrane 14 reduces the amount of substances entering may be 100, 110, 120, 130, 140, or 150 times. In this case, the amount of lactic acid diffusing to enzyme sensing layer 13 is reduced, thereby the sufficient amount of substances of lactic acid oxidase or lactic acid dehydrogenase and other reactants involved in the reaction can be facilitated, and the lactic acid concentration becoming the primary factor limiting the electrode current, resulting in enabling the current magnitude to accurately reflect the lactic acid concentration while significantly increasing the linear range of lactic acid sensor 1.

In some examples, semi-permeable membrane 14 may include first membrane layer 141. First membrane layer 141 may be formed by a first membrane solution. In some examples, a solute of the first membrane solution may include polyvinyl pyridine. In this case, the polyvinyl pyridine is added into first membrane layer 141, the formation of a network structure within first membrane layer 141 can be facilitated, resulting in facilitating the reduction of the entering amount of lactic acid.

In some examples, the first membrane solution may be pure ethanol. In this case, due to the pure ethanol is a good solvent for polyvinyl pyridine and polydimethylsiloxane, the solvent of the first membrane solution is arranged as pure ethanol, thereby the formation of first membrane layer 141 can be facilitated with good morphological consistency, dense morphology, and restricted permeability, resulting in facilitating the improvement of stability of sensor 1.

In some examples, the concentration of polyvinyl pyridine in the first membrane solution may range from 100 mg/mL to 150 mg/mL. For examples, the concentration of polyvinyl pyridine in the first membrane solution may be 100 mg/mL, 110 mg/mL, 120 mg/mL, 130 mg/mL, 140 mg/mL, or 150 mg/mL. Thus, the formation of first membrane layer 141 with permeation-limiting properties can be facilitated, and the improvement of the stability of sensor 1 during continuous monitoring can be further facilitated.

In some examples, the solute of the first membrane solution may further include polydimethylsiloxane. Thus, the formation of first membrane layer 141 with permeation-limiting properties can be facilitated. In some examples, the concentration of polydimethylsiloxane in the first membrane solution may range from 50 mg/mL to 150 mg/mL. For example, the concentration of polydimethylsiloxane may be 50 mg/mL, 60 mg/mL, 80 mg/mL, 100 mg/mL, 120 mg/mL, 130 mg/mL, 140 mg/mL, or 150 mg/mL.

In some examples, the solute of the first membrane solution may further include a first cross-linking agent. In some examples, first membrane layer 141 may be formed by crosslinking the first membrane solution. In some other examples, first membrane layer 141 may be formed by coating enzyme sensing layer 13 with the first membrane solution and then crosslinking it. Thereby, the reduction of amount of lactic acid diffusing to enzyme sensing layer 13 can be facilitated.

In some examples, the first cross-linking agent may be polyethylene glycol diglycidyl ether (PEGDGE400). Thus, it is facilitated that the first membrane solution is cross-linked to form first membrane layer 141.

In some examples, the molecular weight of polyvinyl pyridine in the first membrane solution may range from 10000Da to 500000Da. Thus, the improvement of the membrane-forming properties of first membrane layer 141 can be facilitated.

In some examples, when the molecular weight of polyvinyl pyridine in the first membrane solution ranges from 150000Da to 200000Da, for examples, the molecular weight of polyvinyl pyridine in the first membrane solution may be 150000Da, 160000Da, 170000Da, 180000Da, 190000Da, or 200000Da. In this case, compared to the situation where the molecular weight of polyvinyl pyridine in the first membrane solution ranges from 1 0000Da to 1 00000Da, the molecular weight of polyvinyl pyridine is set within a larger range, the crosslinking time required to form a stable first membrane layer 141 can be reduced and lower the temperature conditions required for crosslinking, resulting in facilitating the improvement of the preparation efficiency of sensor 1 and lower the difficulty of manufacturing sensor 1. In some examples, the first membrane solution may be cross-linked under normal temperature conditions to form first membrane layer 141 with good stability. Wherein, the normal temperature conditions may be referred to 25°C±5°C.

In some examples, the solute of the first membrane solution may further include polyvinyl pyridine-polystyrene copolymer. Thus, the formation of first film layer 141 with limiting effect on permeability can be facilitated.

In some examples, semi-permeable membrane 14 may further include a second membrane layer 142 with biocompatibility. In some examples, the second membrane layer 142 may be formed by a second membrane solution. In some examples, the solute of the second membrane solution may include polyvinyl pyridine-co-polystyrene. In this case, the polyvinyl pyridine-co-polystyrene is added into second membrane layer 142, the improvement of the mechanical strength and biocompatibility of second membrane layer 142 can be facilitated.

In some examples, the concentration of polyvinyl pyridine-co-polystyrene in the second membrane solution may range from 50mg/mL to 150mg/mL. For examples, the concentration of polyvinyl pyridine-co-polystyrene in the second membrane solution may be 50mg/mL, 80mg/mL, 100mg/mL, 120mg/mL, 130mg/mL, 140mg/mL, or 150mg/mL. Thereby, the improvement of the mechanical strength and biocompatibility of second membrane layer 142 can be further facilitated.

In some examples, the solvent of the second membrane solution may be pure ethanol. In this case, the solvent of the second membrane solution is provided to be pure ethanol, the improvement of the morphological consistency of second membrane layer 142 can be facilitated, resulting in facilitating further improvement of the stability of sensor 1. In some examples, the solvent of the second membrane solution may be the same as the solvent of the first membrane solution. In this case, the solvents of both the second membrane solution and first membrane solution are provided to be the same substance (ethanol), the coordination of the second membrane layer and the first membrane layer during cross-linking process to form a stable semi-permeable membrane can be facilitated, resulting in facilitating further improvement of the stability at the interface between the second membrane layer and the first membrane layer.

In some examples, second membrane layer 142 may be provided on first membrane layer 141. In some examples, first membrane layer 141 may be thicker than second membrane layer 142. In some examples, the thickness ratio of first membrane layer 141 to second membrane layer 142 may range from 1.5:1 to 2.5:1. For example, the thickness ratio of first membrane layer 141 to second membrane layer 142 may be 1.5:1, 1.8:1, 2:1, 2.2:1, or 2.5:1. In this case, thereby it is facilitated for first membrane layer 141 and second membrane layer 142 to have relatively matching morphological changes in the process of cross-linking and drying(i.e., the deformation ratios of the two membrane layers are relatively close), resulting in inhibiting the delamination of the two membrane layers during the formation process of semi-permeable membrane 14, which may cause wrinkling or cracking, and further improving the consistency and stability of the morphology of semi-permeable membrane 14 of sensor 1.

In some examples, the thickness of the first membrane layer 141 may range from 30µm to 80µm. For examples, the thickness of first membrane layer 141 may be 30µm, 40µm, 45µm, 50µm, 55µm, 60µm, 65µm, 70µm, 75µm, or 80µm. Thereby it is facilitated to obtain first membrane layer 141 with a restrictive permeation function and good morphological consistency.

In some examples, the thickness of second membrane layer 142 may range from10µm to 40µm. For examples, the thickness of second membrane layer 142 may be 10µm, 15µm, 20µm, 25µm, 26µm, 30µm, 35µm, or 40µm. Thereby it is facilitated to obtain the second membrane layer 142 with good morphological consistency.

In some examples, the solute of the second membrane solution may further include a second cross-linking agent. In some examples, second membrane layer 142 may be formed by cross-linking of the second membrane solution. In some examples, second membrane layer 142 may be formed by coating the second membrane solution onto first membrane layer 141 then cross-linked. In this case, second film layer 142 with biocompatibility is provided on first membrane layer 141, thereby it is facilitated to obtain sensor 1 with biocompatibility.

In some examples, the second cross-linking agent may also be polyethylene glycol diglycidyl ether (PEGDGE400). Thus, the formation of second membrane layer 142 through cross-linking of the second membrane solution can be facilitated.

In some examples, the arrangement of second membrane layer 142 may allow the service life of monitoring probe 10 to be maintained from 1 day to 24 days, for example, it may be 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, or 24 days. Thus, it is facilitated for user to select lactic acid sensors 1 with different service lives based on different needs (such as price, etc.)

In some examples, sensor 1 may further include substrate 11 (see Fig.2). In some examples, substrate 11 may have insulating property. In some examples, substrate 11 may be flexible substrate 11. In some examples, substrate 11 may be flexible insulating substrate 11. In some examples, the material of flexible insulating substrate 11 may be at least one of polyimide (PI), polyethylene terephthalate (PET), Parylene, silicone resin, polydimethylsiloxane (PDMS), polyethylene glycol (PEG), polytetrafluoroethylene resin (Teflon), polyethylene (PE), polypropylene (PP), polystyrene (PS), and polyethylene naphthalate (PEN). Thereby substrate 11 has both flexible and insulating properties, resulting in facilitating the reduction of discomfort after implantation in the body.

In some examples, sensor 1 may further include reference electrode 15 and counter electrode 16 (see Fig.2). Thus, the formation of a stable current circuit can be facilitated. In some examples, working electrode 12, reference electrode 15, and counter electrode 16 may be provided in stack (see Fig.2). Thus, the reduction of the manufacturing difficulty of sensor 1 can be facilitated.

In some examples, working electrode 12 and reference electrode 15 may be stacked on the same side of substrate 11. In some examples, insulating layer 17 may be provided between working electrode 12 and reference electrode 15 (see Fig.2). Thus, the formation of stable current circuit can be facilitated.

The present disclosure is not limited to hereof. In other examples, working electrode 12, reference electrode 15, and counter electrode 16 may be provided on the same plane. That is, working electrode 12, reference electrode 15, and counter electrode 16 may be provided on the same side of substrate 11 in non-stacked manner of each other (see Fig.3). In this case, working electrode 12, reference electrode 15, and counter electrode 16 are provided on the same plane, thereby the thickness of monitoring probe 10 can be reduced, resulting in facilitating the reduction of discomfort after implantation into the body.

In some examples, counter electrode 16 may be made of platinum, silver, silver chloride, palladium, titanium, or iridium. Thus, the electrochemical reaction at working electrode 12 may not be affected under conditions of good conductivity. However, the present embodiment is not limited to hereof. In other examples, counter electrode 16 may also be made of at least one material selected from gold, glassy carbon, graphite, silver, silver chloride, palladium, titanium, or iridium. Thus, the impact on working electrode 12 may be reduced under conditions of good conductivity. In some examples, the same material may be used for working electrode 12, counter electrode 16, and reference electrode 15.

In some examples, known and fixed potential difference may be formed between reference electrode 15 and the tissue fluid or blood. In this case, the potential difference between working electrode 12 and the tissue fluid or blood may be measured through the potential difference formed by reference electrode 15 and working electrode 12, thereby accurately grasping the voltage generated by working electrode 12. Thus, electronic system 20 may automatically adjust and maintain the stability of the voltage at working electrode 12 based on a preset voltage value, resulting in ensuring the measured current signals to accurately reflect the concentration value of lactic acid.

In some examples, when the fluctuation of the potential difference between working electrode 12 and the tissue fluid or blood is not significant, reference electrode 15 may not be used.

In some examples, the current signals generated by monitoring probe 10 may be transmitted to electronic system 20. In this case, the current signals obtained by working electrode 12 is transmitted to electronic system 20 for analyzing, thereby facilitating sensor 1 to obtain the concentration signals of lactic acid.

In some examples, electronic system 20 may transmit the concentration signals of lactic acid to an external reading device via wireless communication methods such as Bluetooth or Wi-Fi, etc. The reading device may receive the concentration signals of lactic acid sent by electronic system 20 and display the concentration value of lactic acid. In addition, continuous monitoring of monitoring probe 10 may be achieved according to the present embodiment, therefore, the purpose of continuously monitoring the human body's lactic acid concentration for an extended period (e.g., from 1 to 24 days) can be achieved. In addition, in some examples, the reading device may be a reader or a mobile phone APP.

In some examples, monitoring probe 10 and electronic system 20 may not be required calibration during in-vivo use. In addition, monitoring probe 10 and electronic system 20 may be pre-calibrated prior to delivery from factory. Thus, the reduction of potential source of errors in the reading of the monitoring module during a user's use can be facilitated.

In some examples, monitoring probe 10 may acquire the lactic acid concentration in the tissue fluid or blood. However, the present disclosure is not limited to hereof. For examples, enzyme sensing layer 13 on monitoring probe 10 may be changed, data on bodily fluid compositions other than lactic acid may also be acquired, for example, the fluid compositions hereof may be glucose, acetylcholine, amylase, bilirubin, cholesterol, chorionic gonadotropin, creatine kinase, creatine, creatinine, DNA, fructosamine, glucose, glutamine, growth hormone, hormones, ketones, lactate, oxygen, peroxides, prostate-specific antigen, prothrombin, RNA, thyroid-stimulating hormone, and troponin.

In some examples, monitoring probe 10 may also monitor the concentration of drugs in body fluids, such as antibiotics (e.g., gentamicin, vancomycin, etc.), digoxin, digitalis, theophylline, and warfarin.

In summary, in the first aspect of the present disclosure, sensor 1 for monitoring lactic acid with high sensitivity and good stability can be provided.

The second aspect of the present disclosure provides a preparation method of sensor 1 for monitoring lactic acid. The preparation method of sensor 1 for monitoring lactic acid in the second aspect of the present disclosure may be referred to as "preparation method of lactic acid monitoring sensor 1", "preparation method of lactic acid sensor 1", " preparation method of sensor 1", or simply "preparation method".

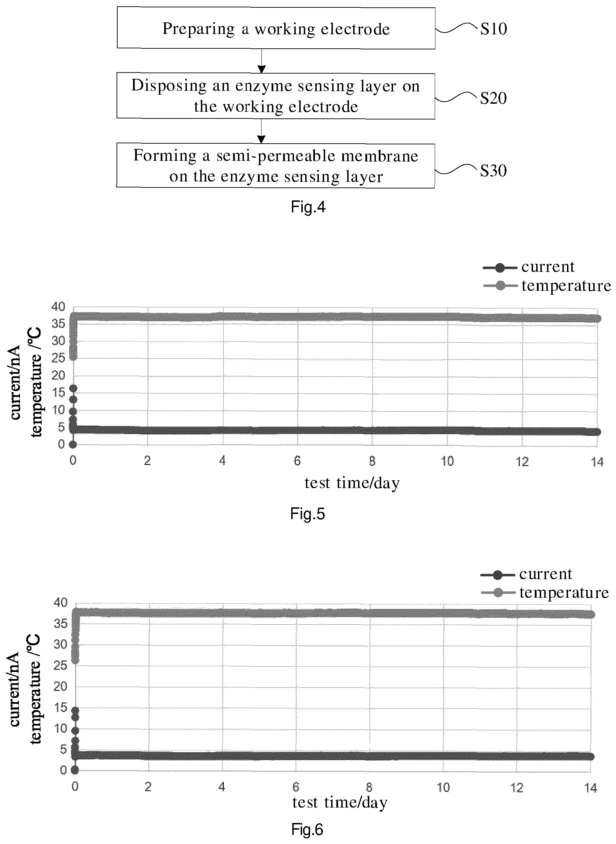

Fig.4 is a flowchart showing the preparation of sensor 1 according to the present disclosure.

The preparation method of present disclosure includes: Preparing working electrode 12, wherein working electrode 12 is formed by a nano slurry, which is a conductive slurry having nanoparticles; Providing enzyme sensing layer 13 on working electrode 12, wherein enzyme sensing layer 13 includes lactic acid enzyme and is capable of reacting with lactic acid; Forming semi-permeable membrane 14 on enzyme sensing layer 13, semi-permeable membrane 14 at least covers enzyme sensing layer 13 and includes first membrane layer 141 formed by a first membrane solution, wherein the solute of the first membrane solution includes polyvinyl pyridine and the solvent is pure ethanol. Sensor 1 prepared from the preparation method according to the second aspect of the present disclosure is consistent with sensor 1 according to the first aspect of the present disclosure, the specific descriptions of sensor 1 may refer to the above descriptions of sensor 1 and will not be repeated hereof.

In the second aspect of the present disclosure, the preparation method is used to prepare sensor 1 for monitoring lactic acid, thereby sensor 1 with high sensitivity and good stability for monitoring lactic acid can be obtained.

Hereinafter, sensor 1 for monitoring lactic acid provided in the present disclosure will be described in detail with reference to embodiments and comparative examples, but they should not be construed as limiting the scope of protection of the present disclosure.

### [Embodiments]

### [Embodiment 1-3]

Firstly, preparing a monitoring probe with a working electrode.

Preparing lactic acid-sensitive solution according to Table 1, depositing the lactic acid-sensitive solution on the working electrode to form 13 discrete points, the diameter of each point is 120±30µm, resulting in obtaining the working electrode with an enzyme sensing layer after curing overnight.

Preparing the first membrane solution according to Table 1, coating the first membrane solution on the working electrode with an enzyme sensing layer and cross-linked for 1 hour in an environment with a temperature of 25°C and a relative humidity of 60%, resulting in obtaining the first membrane layer with thickness shown in Table 1.

Preparing the second membrane solution according to Table 1, coating the second membrane solution on the working electrode with the first membrane layer and cross-linked for 24 hours in an environment with temperature of 25°C and a relative humidity of 60%, resulting in obtaining the second membrane layer with thickness shown in in Table 1; the sensors of Embodiment1 to Embodiment 3 are obtained.

**Table1**

| | | Lactic acid-sensitive solution | | The first membrane solution | | The second membrane solution | | The thickness of the first membrane layer ad the second membrane layer |
|---|---|---|---|---|---|---|---|---|
| | | Substance | Concent ration (mg/m L) | Substance | Concent ration (mg/m L) | Substance | Concent ration (mg/m L) | |
| Emb odim ent 1 | Sol ute | Lactic acid oxidase | 24.6 | Polyvinyl pyridine | 100 | Polyvinyl pyridine-styrene copolymer | 120 | The first membrane layer 65µm, the second membrane layer 26µm, the thickness ratio is 2.5:1 |
| | | Human serum albumin | 24.6 | Polydimethyl siloxane | 3.5 | PEGDGE4 00 | 40 | |
| | | Metal polymer (Osmium bipyridyl complex coordinated to poly(4-vinylpyridine)) | 20 | PEGDGE40 0 | 30 | \ | \ | |
| | | PEGDGE400 | 6.2 | \ | \ | \ | \ | |
| | Sol ven t | 10 mmol/L 4-Morpholineethanes ulfonic acid solution | \ | Pure ethanol | \ | Pure ethanol | \ | |
| Emb odim ent 2 | Sol ute | Lactic acid oxidase | 24.6 | Polyvinyl pyridine | 150 | Polyvinyl pyridine-styrene copolymer | 120 | The first membrane layer 65µm, the second membrane layer 26µm, the thickness ratio is 2.5:1 |
| | | Human serum albumin | 24.6 | Polydimethyl siloxane | 3.5 | PEGDGE4 00 | 40 | |
| | | Metal polymer (Osmium bipyridyl complex coordinated to poly(4-vinylpyridine)) | 20 | PEGDGE40 0 | 30 | \ | \ | |
| | | PEGDGE400 | 6.2 | \ | \ | \ | \ | |
| | Sol ven t | 10 mmol/L 4-Morpholineethanes ulfonic acid solution | \ | Pure ethanol | \ | Pure ethanol | \ | |
| Emb odim ent 3 | Sol ute | Lactic acid oxidase | 24.6 | Polyvinyl pyridine | 100 | Polyvinyl pyridine-styrene copolymer | 120 | The first membrane layer 65µm, the second membrane layer 26µm, the thickness ratio is 2.5:1 |
| | | Human serum albumin | 24.6 | Polydimethyl siloxane | 3.5 | PEGDGE4 00 | 40 | |
| | | Metal polymer (Osmium bipyridyl complex coordinated to poly(4-vinylpyridine)) | 20 | PEGDGE40 0 | 30 | \ | \ | |
| | | PEGDGE400 | 6.2 | polyvinyl pyridine-polystyrene copolymer | 140 | \ | \ | |
| | Sol ven t | 10 mmol/L 4-Morpholineethanes ulfonic acid solution | \ | Pure ethanol | \ | Pure ethanol | \ | |

The sensors from Embodiment 1 to Embodiment 3 were exposed to 5mM lactic acid solution in 500mM phosphate buffered saline (PBS) at 37±1°C for 336 hours (14 days), wherein the working potential is maintained at +50mV relative to Ag/AgCl.

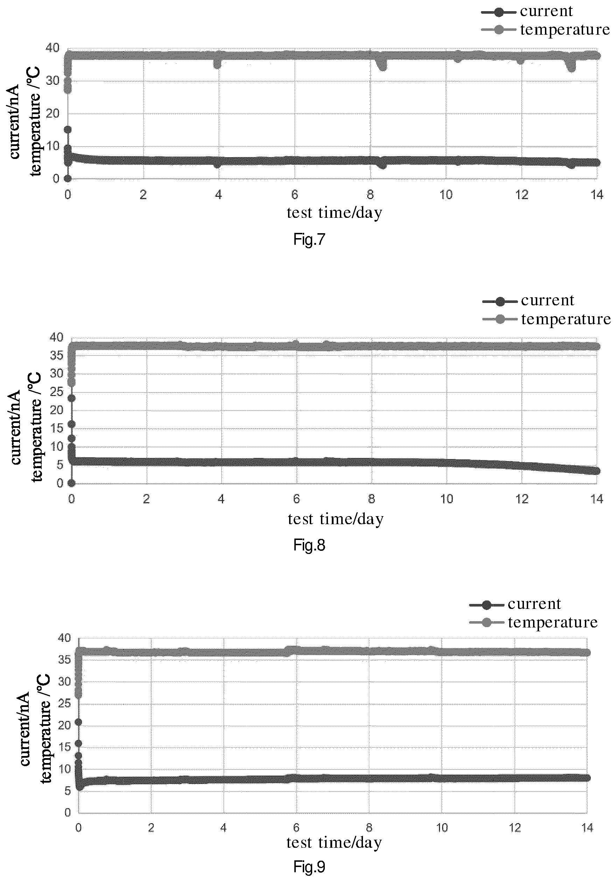

Fig.5 is a graph showing the current and temperature response-test time of the sensor according to Embodiment 1 of the present disclosure. Fig.6 is a graph showing the current and temperature response-test time of the sensor according to Embodiment 2 of the present disclosure. Fig.7 is a graph showing the current and temperature response-test time of the sensor according to Embodiment 3 of the present disclosure.

It may be seen from Fig.5 that during the testing period from day 0 to day 14, the current signals of the sensor are approximately 5nA and remain relatively stable, without obvious attenuation, at the same time, the sensitivity of the sensor's current signals against to lactic acid concentration is approximately 1nA/mM, indicating high sensitivity.

It may be seen From Fig.6 that during the testing period from day 0 to day 14, the current signals of the sensor are approximately 4nA and remain relatively stable, without obvious attenuation, at the same time, the sensitivity of the sensor's current signals against to lactic acid concentration is approximately 0.8nA/mM. Compared to Embodiment 1, the concentration of polyvinyl pyridine in the first membrane solution was increased from 100mg/mL to 150mg/mL in Embodiment 2. Based on the test results, both sensors in Embodiment 2 and Embodiment 3 show good stability over a 14-day testing period, meanwhile, the current signal generated by the sensor in Embodiment 1 is 5nA, while the current signal generated by the sensor in Embodiment 2 is 4nA, the current signal generated in Embodiment 1 is slightly higher than that generated in Embodiment 2.

It may be seen From Fig.7 that during the testing period from day 0 to day 14, the current signals of the sensor are approximately 6nA and remain relatively stable, without obvious attenuation. At the same time, the sensitivity of the sensor's current signals against to lactic acid concentration is approximately 1.2nA/mM, indicating a high sensitivity. It may also be seen from Fig.7 that there are three slight fluctuations in the current values during the testing period from day 0 to day 14. By observing the relationship between the current curve and the temperature curve in Fig.7, it may be determined that the fluctuation in current values is caused by temperature changes of the testing environment.

### [Comparative Examples]

### Comparative Example 1

Compared to Embodiment 1, only the concentration of polyvinyl pyridine in the first membrane solution is adjusted to 50mg/mL, while the rest is consistent with Embodiment 1, resulting in obtaining the sensor of Comparative Example 1.

The sensor of Comparative Example 1 is exposed to a 5mM lactic acid solution in 500mM phosphate buffered saline (PBS) at 37±1 °C for 336 hours (14 days), with the working potential being maintained at +50mV relative to Ag/AgCl. Fig.8 is a graph showing the current and temperature response-test time of the sensor according to Comparative Example 1 of the present disclosure. As may be seen from Fig8 that during the testing period from day 0 to day 10, the current signals of the sensor are approximately 6nA and remain relatively stable, without obvious attenuation, at the same time, the sensitivity of the sensor's current signals against to lactic acid concentration is approximately 1.2nA/mM, indicating a high sensitivity. However, during the testing period from day 10 to day 14, the current signals gradually attenuate, on day 14, the current signal of the sensor is approximately 4nA, with the sensitivity being decreased to 0.8nA/mM. It may been be seen thereof, the concentration of polyvinyl pyridine in the first membrane solution is set to be above 100mg/mL, it is facilitated for the sensor to be maintained in good stability throughout the testing period.

### Comparative Example 2

Compared to Embodiment 1, only the solvent in the first membrane solution is adjusted to a ratio of 8:2 of the ethanol: HEPES buffer solution, while the rest is consistent with Embodiment 1, resulting in obtaining the sensor of Comparative Example 2.

The sensor of Comparative Example 2 is exposed to a 5mM lactic acid solution in 500mM phosphate buffered saline (PBS) at 37±1°C for 336 hours (14 days), with the working potential being maintained at +50mV relative to Ag/AgCl. Fig.9 is a graph showing the current and temperature response-test time of the sensor according to Comparative Example 2 of the present disclosure. It may be seen from Fig.9, in overall that, during the testing period from day 0 to day14, the current signals of the sensor remain relatively stable. Wherein, from day 0 to day 6, the current signals of the sensor are approximately 7nA, with a sensitivity of approximately 1.4nA/mM, from day 6 to day 14, the current signals of the sensor are approximately 8nA, with a sensitivity of approximately 1.6nA/mM. By observing the relationship between the current curve and the temperature curve in Fig.9, it may be determined that the fluctuation in the current values is caused by temperature changes of the testing environment.

Fig.10 is a photograph showing the monitoring probe of the sensor according to Comparative Example 2 of the present disclosure. Fig.11 is another photograph showing different view of the monitoring probe of the sensor according to Comparative Example 2 of the present disclosure. It may be seen from Fig.10 and Fig.11 that, when ethanol: HEPES buffer solution with a volume ratio of 8:2 is used as the solvent to prepare the first membrane layer, the morphology of the resulting semi-permeable membrane shrinks, which is not facilitated to improve the consistency and stability of the semi-permeable membrane. Fig.12 is a photograph showing the monitoring probe of the sensor according to Embodiment 1 of the present disclosure. By comparison, the morphological consistency of the semi-permeable membrane obtained in Embodiment 1 of Fig.12 (using pure ethanol as the solvent for preparing the first membrane layer) is obviously better, which is facilitated to improve the stability of the semi-permeable membrane.

### Comparative Example 3

Preparing a monitoring probe with a working electrode in the same manner as Embodiment 1, and providing an enzyme sensing layer consistent with Embodiment 1 on the working electrode, resulting in obtaining a sensor without disposing semi-permeable membrane.

The sensor of Comparative Example 3 is placed in a 0.5 mM lactic acid solution for measurement, and the measured current signal is 27.5 nA. The sensors of Embodiment 1, Embodiment 2, and Embodiment 3 are respectively placed in 0.5 mM lactic acid solution for measurement, the current signal measured by the sensor of Embodiment 1 is 1.0 nA, and the permeability of the semi-permeable membrane in Embodiment 1 is calculated to be approximately 3.64% according to the formula (permeability = current signal of Embodiment 1 / current signal of Comparative Example 3 * 100%); The current signal measured by the sensor of Embodiment 2 is 0.8 nA, in the same way, it may be seen that the permeability of the semi-permeable membrane in Embodiment 2 is approximately 2.91%; The current signal measured by the sensor of Embodiment 3 is 1.2 nA, in the same way, it may be seen that the permeability of the semi-permeable membrane in Embodiment 3 is approximately 4.36%. Therefore, it can be proved that the semi-permeable membranes in each embodiment can play a role in reducing the amount of lactic acid entering.

The sensor of Comparative Example 3 is exposed to a 5 mM lactic acid solution in 500 mM phosphate buffered saline (PBS) at 37±1°C for 2.5 hours, wherein the working potential is maintained at +50 mV relative to Ag/AgCl. Fig.13 is a graph showing the current and temperature response-test time of the sensor according to Comparative Example 3 of the present disclosure. As shown in Fig.13 that during the 140-minute testing time, the current signals generated by the sensor gradually decrease from 120 nA to less than 10 nA, indicating poor stability. The test result indicates that the sensor without semi-permeable membrane shows poor stability when the concentration of lactic acid solution is measured.

### Comparative Example 4

Compared to Embodiment 1, only the concentration of metal polymer in the lactic acid-sensitive solution is adjusted to 9.2 mg/mL, while the rest is consistent with Embodiment 1, resulting in obtaining the sensor of Comparative Example 4.

The sensor of Comparative Example 4 is exposed to a 5 mM lactic acid solution in 500 mM phosphate buffered saline (PBS) at 37±1°C for 336 hours (14 days), wherein the working potential is maintained at +50 mV relative to Ag/AgCl. Fig.14 is a graph showing the current and temperature response-test time of the sensor according to Comparative Example 4 of the present disclosure. As shown in Fig.4 that during the test time from day 0 to day 14, the current signals of the sensor gradually attenuate, in the initial testing phase, the current signals are approximately 9 nA, and on day 14, the current signals approach 0 nA. By comparing the Embodiment 1 with Comparative Example 4, it is found that the concentration of metal polymer in the lactic acid-sensitive solution in Embodiment 1 is 20 mg/mL, while the concentration of metal polymer in the lactic acid-sensitive solution in Comparative Example 4 is 9.2 mg/mL. It may be seen thereof, the metal polymer concentration in the lactic acid sensitive solution is prepared as 20mg/mL, which is facilitated for the sensor to maintain good stability in the testing time from day 1 to day 14.

### Comparative Example 5

Compared to Embodiment 1, the difference is that only the thickness ratio of the first membrane layer to the second membrane layer is adjusted to 1:1, the thickness of the first membrane layer is 26 µm. The rest is consistent with Embodiment 1, resulting in obtaining the sensor of Comparative Example 5.

The sensor of Comparative Example 5 is exposed to a 5 mM lactic acid solution in 500 mM phosphate buffered saline (PBS) at 37±1°C for 336 hours (14 days), wherein the working potential is maintained at +50 mV relative to Ag/AgCl. Fig.15 is a graph showing the current and temperature response-test time of the sensor according to Comparative Example 5 of the present disclosure. As shown in Fig.15, during the testing time from day 0 to day 12, the current signals of the sensor are relatively stable, the current values are approximately 7 nA and the sensitivity is approximately 1.4 nA/mM. From day 12 to day 14, the current signals gradually attenuate. On day 14, the current value is approximately 6 and the sensitivity is approximately 1.6 nA/mM. Comparing the Embodiment 1 with Comparative Example 5, it may be seen that the thickness of the first membrane layer in Embodiment 1 is 65 µm, the thickness of the first membrane layer in Comparative Example 5 is 26 µm, the thickness of the second membrane layer in both Embodiment 1 and Comparative Example 5 is 26 µm (the thickness ratio of the first membrane layer to the second membrane layer in Embodiment 1 is 2.5:1, the thickness ratio of the first membrane layer to the second membrane layer in Embodiment Comparative Example 5 is 1:1). It may be seen thereof, the thickness ratio of the first membrane layer to the second membrane layer is adjusted to 2.5:1, which is facilitated to improve the stability of the sensor.

### Comparative Example 6

Compared to Embodiment 1, the difference is that only the thickness ratio of the first membrane layer to the second membrane layer is adjusted to 3:1, the thickness is 78 µm, the rest was consistent with Embodiment 1, resulting in obtaining the sensor of Comparative Example 6.

The sensor of Comparative Example 6 is exposed to a 5 mM lactic acid solution in 500 mM phosphate buffered saline (PBS) at 37±1°C for 336 hours (14 days), wherein the working potential is maintained at +50 mV relative to Ag/AgCl. Fig.16 is a graph showing the current and temperature response-test time of the sensor according to Comparative Example 6 of the present disclosure. As shown in Fig.16, during the testing time from day 0 to day 14, the current signals of the sensor are approximately 5 nA and remain relatively stable, without obvious attenuation, and the sensitivities are approximately 1 nA/mM. Fig.17 is a photograph showing the monitoring probe of the sensor according to Comparative Example 6 of the present disclosure. It may be seen that the morphology of the semi-permeable membrane prepared in Comparative Example 6 is wrinkled (see Fig.17), indicating poor consistency and stability for the semi-permeable membrane. By comparing the Embodiment 1 with Comparative Example 6, it may be seen that the thickness ratio of the first membrane layer to the second membrane layer in Embodiment 1 is 2.5:1, the thickness ratio of the first membrane layer to the second membrane layer in Comparative Example 1 is 3:1. It may be see thereof, the thickness ratio of the first membrane layer to the second membrane layer is prepared as 2.5:1, which is facilitated for the first membrane layer and the second membrane layer to match the morphological changes in the process of cross-linking and drying (that is, the deformation ratio of the two membrane layers is close). Thereby, the shrinking or cracking caused by the delamination of the two membrane layers in the formation process of the semi-permeable membrane can be inhibited, the improvement of the consistency and stability of the semi-permeable membrane morphology of the sensor can be further facilitated.

In summary, the sensors of each embodiment (Embodiment 1 to Embodiment 3) showed high sensitivity and excellent stability during the testing period from day 0 to day 14, and the consistency in the semi-permeable membrane morphology are good. Relatively speaking, the sensors in the comparative examples (Comparative Examples 1 to 6) cannot simultaneously achieve the performance in sensitivity, stability, and consistency in the semi-permeable membrane morphology comparable to those of the sensors in the embodiments (Embodiment 1 to Embodiment 3).

Although the present disclosure has been described in detail with reference to the drawings and examples, it should be understood that the above descriptions do not intend to limit the disclosure in any form. A skilled person in the art may make modifications and variations to the disclosure as needed without deviating from the essential spirit and scope of the disclosure, and such modifications and variations fall within the scope of the disclosure.

## Claims

1. A sensor for monitoring the lactic acid, **characterized in that**,
The sensor includes a working electrode, an enzyme sensing layer, and a semi-permeable membrane,
The working electrode is formed by a nano slurry, the nano slurry is a conductive slurry having nanoparticles,
The enzyme sensing layer is provided on the working electrode, the enzyme sensing layer includes a lactic acid enzyme and is capable of reacting with lactic acid,
The semi-permeable membrane is provided on the enzyme sensing layer and at least covers the enzyme sensing layer, the semi-permeable membrane includes a first membrane layer formed by a first membrane solution, wherein a solute of the first membrane solution includes polyvinyl pyridine and a solvent is pure ethanol.

2. The sensor according to claim 1, **characterized in that**,
The enzyme sensing layer is formed by a lactic acid-sensitive solution, the lactic acid-sensitive solution includes lactic acid enzyme, serum albumin, metal polymer, and crosslinking agent.

3. The sensor according to claim 2, **characterized in that**,
The metal polymer is selected from at least one of poly(vinylferrocene), quaternary ammonium poly(4-vinylpyridine) of ferricyanide, quaternary ammonium poly(1-vinylimidazole) of ferricyanide, quaternary ammonium poly(4-vinylpyridine) of ferrocyanide, quaternary ammonium poly(1-vinylimidazole) of ferrocyanide, osmium bipyridyl complex coordinated to poly(1-vinylimidazole), osmium bipyridyl complex coordinated to poly(4-vinylpyridine), cobalt bipyridyl complex coordinated to poly(1-vinylimidazole), or cobalt bipyridyl complex coordinated to poly(4-vinylpyridine).

4. The sensor according to claim 2, **characterized in that**,
The lactic acid-sensitive solution is drop-coated onto the working electrode in the form of a plurality of sensing spots to form the enzyme sensing layer, diameters of the sensing spots range from 80µm to 170µm, and the number of the sensing spots ranges from 6 to15.

5. The sensor according to claim 1, **characterized in that**,
A concentration of the polyvinyl pyridine in the first membrane solution ranges from 100mg/mL to 150mg/mL.

6. The sensor according to claim 1, **characterized in that**,
A molecular weight of the polyvinyl pyridine ranges from 10000Da to 500000Da.

7. The sensor according to claim 1, **characterized in that**,
The semi-permeable membrane further includes a second membrane layer having biocompatibility, the second membrane layer is formed by a second membrane solution, a solute of the second membrane solution includes polyvinyl pyridine -co-polystyrene, and a solvent is pure ethanol.

8. The sensor according to claim 7, **characterized in that**,
A concentration of polyvinyl pyridine-co-polystyrene in the second membrane solution ranges from 50mg/mL to 150mg/mL.

9. The sensor according to claim 7, **characterized in that**,
The second membrane layer is provided on the first membrane layer, and a thickness ratio of the first membrane layer with the second membrane layer ranges from 1.5:1 to 2.5:1.

10. A preparation method of the sensor for monitoring lactic acid, **characterized in that**, the preparation method includes:
Preparing a working electrode, wherein the working electrode is formed by a nano slurry, the nano slurry is a conductive slurry having nanoparticles.
Providing an enzyme sensing layer on the working electrode, wherein the enzyme sensing layer includes lactic acid enzyme and is capable of reacting with lactic acid.
Forming a semi-permeable membrane on the enzyme sensing layer, the semi-permeable membrane at least covers the enzyme sensing layer and includes a first membrane layer formed by a first membrane solution, wherein a solute of the first membrane solution includes polyvinyl pyridine and a solvent is pure ethanol.
